# EUROPEAN PATENT SPECIFICATION

(11) **EP 2 589 302 B1**
(45) Date of publication and mention of the grant of the patent: **23.11.2016**
(21) Application number: 13153122.0
(22) Date of filing: 19.12.2008
(51) Int. Cl.: A61K 31/133, A61K 31/688, A61K 31/702, A61K 31/733, A23L 33/21, A23L 33/10

(54) **Use of sphingomyelin and non-digestible carbohydrates for improving intestinal microbiota**
Verwendung von Sphingomyelin und unverdaulichen Kohlenhydraten zur Verbesserung der Darm-Mikrobiota
Utilisation de shingomyéline et de glucides non digestibles pour améliorer le microbiote intestinal

(30) Priority: 21.12.2007 EP 07124007; 31.03.2008 US 40788 P; 31.03.2008 EP 08153775
(43) Date of publication of application: 08.05.2013
(62) Divisional of application: 08865460.3
(73) Proprietor: N.V. Nutricia, 2712 HM Zoetermeer (NL)
(72) Inventor: Nieuwenhuizen, Willem Ferdinand, 3981 AM Bunnik (NL); Ben Amor, Kaouther, 3511 PH Utrecht (NL); Knol, Jan, 6708 LZ Wageningen (NL); van der Beek, Eline Marleen, 6705 DB Wageningen (NL); Beermann, Christopher, DE-36100 Petersberg (DE); Boehm, Günther, DE-61209 Echzell (DE)
(74) Representative: Nederlandsch Octrooibureau

(56) References cited:
- WO-A-2004/064819
- WO-A1-2004/052121
- WO-A1-2005/035781
- DE-A1- 19 836 339
- SPRONG R C ET AL: "BOVINE MILK FAT COMPONENTS INHIBIT FOOD-BORNE PATHOGENS", INTERNATIONAL DAIRY JOURNAL, ELSEVIER APPLIED SCIENCE, BARKING, GB, vol. 12, no. 2-3, 1 January 2002 (2002-01-01), pages 209-215, XP008075177, ISSN: 0958-6946
- DATABASE WPI Week 200128 Thomson Scientific, London, GB; AN 2001-268278 XP002522263, & JP 2000 350563 A (SNOW BRAND MILK PROD CO LTD) 19 December 2000 (2000-12-19)
- KLEESSEN BRIGITTA ET AL: "Oligofructose and long-chain inulin: Influence on the gut microbial ecology of rats associated with a human faecal flora", BRITISH JOURNAL OF NUTRITION, vol. 86, no. 2, August 2001 (2001-08), pages 291-300, XP002516707, ISSN: 0007-1145
- DJOUZI ZAKIA ET AL: "Compared effects of three oligosaccharides on metabolism of intestinal microflora in rats inoculated with a human faecal flora", BRITISH JOURNAL OF NUTRITION, vol. 78, no. 2, 1997, pages 313-324, XP002516708, ISSN: 0007-1145
- MITSUOKA T ET AL: "Effect of fructo-oligosaccharides on intestinal microflora.", DIE NAHRUNG 1987, vol. 31, no. 5-6, 1987, pages 427-436, XP002516709, ISSN: 0027-769X

## Description

### FIELD OF THE INVENTION

The present invention is in the field of nutritional compositions for restoring or maintaining a well balanced intestinal microbiota. The composition is especially suitable for infants and toddlers.

### BACKGROUND OF THE INVENTION

At birth infants are devoid of an intestinal microbiota. After birth the large intestine is quickly colonised and its microbiota rapidly develops and increases. The intestinal microbiota of breast-fed infants is primarily composed of species belonging to the genus *Bifidobacterium* and other lactic acid producing bacteria. The microbiota of formula-fed infants is more diverse and contains in general more species belonging to *Bacteroides, Clostridium* and *Enterobacteriaceae.* This difference in microbiota composition is alleged to result in many beneficial effects regarding the immune system, the gastro-intestinal barrier function and anti-pathogenic function, resulting in less gastro-intestinal infections in terms of both incidence and duration, less atopic diseases such as food allergy, atopic dermatitis, allergy induced asthma, and less constipation in breast-fed infants.

WO 2006/091103 discloses a composition comprising probiotics and two non-digestible saccharides for the use against several disorders. WO 00/08948 discloses mixtures of non-digestible oligosaccharides and polysaccharides in order to improve the intestinal microbiota.

WO 2006/041316 discloses the use of sphingomyelin degradation products to decrease the amount of *Clostridium difficile.*

WO 2007/073194 discloses the use of compositions comprising phospholipids, sphingolipids and cholesterol and optionally non-digestible carbohydrates for use in prevention of obesity and/or diabetes.

### SUMMARY OF THE INVENTION

The present invention is based on an unexpected advantageous effect of a composition comprising both sphingophospholipid and/or degradation products thereof, in particular ceramide, sphingoid base, sphingoid phosphate and/or lysosphingphospholipid, and non-digestible oligosaccharides on the composition and/or activity of the intestinal microbiota. This combination surprisingly showed an enhanced favourable effect on the composition of the intestinal microbiota. In particular a decreased ratio of Firmicutes/Bacteroidetes, a decreased ratio *Clostridium difficile*/*total* bacteria, and/or an increased prebiotic index was observed.

The inventors recognized that the composition of the microbiota formed during infancy affects the composition of the microbiota later in life. Differences in microbiota development during infancy are considered to result in differences in the composition of intestinal microbiota in children of at least seven years old. The development of a well-balanced microbiota during infancy therefore has health consequences extending beyond infancy, into childhood, adolescence and/or adulthood.

Hence the use of compositions comprising sphingophospholipid and/or degradation products thereof together with non-digestible carbohydrates, will, when administered early in life to infants and/or toddlers with an age below 36 months of age, beneficially affect the prevention of disorders, associated with a imbalanced intestinal microbiotia, for periods extending beyond the period when this composition is administered, i.e. above 36 months of age, such as childhood, adolescence and/or adulthood.

It is thus considered that a well-balanced microflora, especially a microflora with a low ratio of Firmicutes/Bacteroidetes or *Clostridia*/*Bacteroides,* affects the fat storage in adipocytes and/or has a preventive and/or therapeutic effect on obesity. Therefore, the present composition is particularly suitable for treating and/or preventing obesity. Since adipocytes, especially visceral adipocytes, are formed and proliferate during infancy, the present composition is especially suitable for administration to human subjects with an age below 36 months for prevention of obesity when said human subject has reached an age above 36 month of age.

Furthermore, due to the advantageous effect the combination of sphingophospholipids and/or degradation product(s) thereof with non-digestible carbohydrate is particularly advantageous for use in treatment and/or prevention of disorders related to the intestinal microbiota composition, such as gastro-intestinal infections, diarrhoea, gastro-intestinal inflammation and/or constipation.

Since a well balanced intestinal microbiota also improves the immune system and/or increases gut maturation and/or the gastro-intestinal barrier function the present combination also can be used for treatment and/or prevention of atopic diseases such as allergy, more particularly food allergy, atopic dermatitis, allergy induced asthma, and systemic and respiratory infections.

### DETAILED DESCRIPTION OF THE INVENTION

The present invention concerns a method for normalising the intestinal microbiota in a human subject towards the microbiota found in healthy lean subjects and/or to breast-fed infants, said method comprising administering a composition comprising
A sphingophospholipid, preferably sphingomyelin, and/or at least one sphingophospholipid degradation product selected from the group consisting of ceramide, lysosphingophospholipid, sphingoid phosphate, and free sphingoid base, and
B at least one non-digestible carbohydrate selected from the group consisting of fructo-oligosaccharides, galacto-oligosaccharides, gluco-oligosaccharides, arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides
to said human subject.

In other words, the present invention concerns the use of a nutritional composition comprising
A sphingophospholipid, preferably sphingomyelin, and/or at least one sphingophospholipid degradation product selected from the group consisting of ceramide, lysosphingophospholipid, sphingoid phosphate, and free sphingoid base, and
B at least one non-digestible carbohydrate selected from the group consisting of fructo-oligosaccharides, galacto-oligosaccharides, gluco-oligosaccharides, arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides
for the manufacture of a composition for normalising the intestinal microbiota in a human subject towards the microbiota found in healthy lean subjects and/or to breast-fed infants.

The present the present invention can also be worded as a nutritional composition comprising
A sphingophospholipid, preferably sphingomyelin, and/or at least one sphingophospholipid degradation product selected from the group consisting of ceramide, lysosphingophospholipid, sphingoid phosphate, and free sphingoid base, and
B at least one non-digestible carbohydrate selected from the group consisting of fructo-oligosaccharides, galacto-oligosaccharides, gluco-oligosaccharides, arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides
for normalising the intestinal microbiota in a human subject towards the microbiota found in healthy lean subjects and/or to breast-fed infants.

### Non-digestible carbohydrates

The present composition comprises non-digestible carbohydrates. Non-digestible carbohydrates improve the microbiota. Non-digestible carbohydrates stimulate the growth and/or activity of bifidobacteria and bacteroides. As is shown in example 1, administration of non-digestible carbohydrates was found to advantageously decrease the ratio of Firmicutes/Bacteroidetes, i.e. clostridia/bacteroides.

Preferably, the present composition comprises non-digestible carbohydrate with a DP between 2 and 250, more preferably 2 to 60. The non-digestible carbohydrate is at least one, preferably at least two, selected from the group consisting of fructo-oligosaccharides (including inulin), galacto-oligosaccharides (including transgalacto- oligosaccharides or beta-galacto-oligosaccharides), gluco-oligosaccharides (including gentio-, nigero- and cyclodextrin-oligosaccharides and polydextrose), arabino-oligosaccharides, mannan-oligosaccharides, xylo- oligosaccharides, fuco-oligosaccharides, arabinogalacto-oligosaccharides (including gum acacia), glucomanno-oligosaccharides, galactomanno-oligosaccharides (including partially hydrolysed guar gum), sialic acid comprising oligosaccharides and uronic acid oligosaccharides (including galacturonic acids and pectin degradation products). Preferably the present composition comprises fructo-oligosaccharides, galacto-oligosaccharides and/or galacturonic acid oligosaccharides, more preferably galacto-oligosaccharides, most preferably betagalacto-oligosaccharides.

Preferably the composition comprises galacto-oligosaccharides. The galacto-oligosaccharide is preferably selected from the group consisting of beta-galacto-oligosaccharides, lacto-N-tetraose (LNT), lacto-N-neotetraose (neo-LNT), fucosyl-lactose, fucosylated LNT and fucosylated neo-LNT. In a particularly preferred embodiment the present composition comprises beta-galacto-oligosaccharides. Beta-galacto-oligosaccharides as used in the present invention refers to oligosaccharides composed of over 50%, preferably over 65% galactose units based on monomeric subunits, with a degree of polymerization (DP) of 2 to 20, in which at least 50%, more preferably at least 75%, even more preferably at least 90%, of the galactose units are linked together via a beta-glycosidic linkage, preferably a beta-1,4 glycosidic linkage. The average DP is preferably of 3 to 6. A glucose unit may be present at the reducing end of the chain of galactose units. Beta-galacto-oligosaccharides are sometimes also referred to as transgalacto-oligosacchariodes (TOS). A suitable source of beta-galacto-ologosaccharides is Vivinal®GOS (commercially available from Borculo Domo Ingredients, Zwolle, Netherlands). Other suitable sources are Oligomate (Yakult), Cupoligo, (Nissin) and Bi2muno (Classado).

Preferably the composition comprises fructo-oligosaccharides. Fructo-oligosaccharide as used in the present invention refers to carbohydrates composed of over 50%, preferably over 65 % fructose units based on monomeric subunits, in which at least 50%, more preferably at least 75%, even more preferably at least 90%, of the fructose units are linked together via a beta-glycosidic linkage, preferably a beta-2,1 glycosidic linkage. A glucose unit may be present at the reducing end of the chain of galactose units. Preferably the fructo-oligosaccharide has a DP or average DP of 2 to 250, more preferably 2 to 100, even more preferably 10 to 60. Fructo-oligosaccaride comprises levan, hydrolysed levan, inulin, hydrolysed inulin, and synthesised fructo-oligosaccharides. Preferably the composition comprises short chain fructo-oligosaccharides with an average degree of polymerization (DP) of 3 to 6, more preferably hydrolysed inulin or synthetic fructo-oligosaccharide. Preferably the composition comprises long chain fructan with an average DP above 20, such as RaftilinHP. Preferably the composition comprises both short chain and long chain fructan. Fructo-oligosaccharide suitable for use in the compositions is also readily commercially available, e.g. RaftilineHP (Orafti).

The present composition preferably comprises uronic acid oligosaccharides, more preferably galacturonic acid oligosaccharides. The term galacturonic acid oligosaccharide as used in the present invention refers to an oligosaccharide wherein at least 50% of the monosaccharide units present in the oligosaccharide is galacturonic acid. The galacturonic acid oligosaccharides used in the invention are preferably prepared from degradation of pectin, pectate, and/or polygalacturonic acid. Preferably the degraded pectin is prepared by hydrolysis and/or beta-elimination of fruit and/or vegetable pectins, more preferably apple, citrus and/or sugar beet pectin, even more preferably apple, citrus and/or sugar beet pectin degraded by at least one lyase. In a preferred embodiment, at least one of the terminal galacturonic acid units of the galacturonic acid oligosaccharide has a double bond. The double bond effectively protects against attachment of pathogenic bacteria to intestinal epithelial cells. Preferably one of the terminal galacturonic acid units comprises a C₄-C₅ double bond. The galacturonic acid oligosaccharide can be derivatised. The galacturonic acid oligosaccharide may be methoxylated and/or amidated. In one embodiment the galacturonic acid oligosaccharides are characterised by a degree of methoxylation above 20%, preferably above 50% even more preferably above 70%.

In a preferred embodiment the composition comprises a mixture of inulin and fructo-oligosaccharides. In a preferred embodiment the composition comprises a mixture of galacto- oligosaccharides and a fructo-oligosaccharides selected from the group consisting of short chain fructo-oligosaccharides and inulin, more preferably inulin. A mixture of at least two different non-digestible carbohydrates advantageously stimulates the beneficial bacteria of the intestinal microbiota to a greater extent than based on the single non-digestible carbohydrates. Preferably the weight ratio between the mixture of two different non-digestible carbohydrates, preferably galacto-oligosaccharides and fructo-oligosaccharide, is between 20 and 0.05, more preferably between 20 and 1. Galacto-oligosaccharides are more reminiscent to the human milk oligosaccharides. Preferably the present composition comprises galacto-oligosaccharides with a DP of 2-10 and/or fructo-oligosaccharides with a DP of 2-60. These combination was found to synergistically increase bifidobacteria and lactobacilli. Preferably the composition comprises beta-galacto-oligosaccharide, fructo-oligosaccharide and a pectin degradation product. The presence of these three non-digestible oligosaccharides even further stimulates the bifidobacteria, while decreasing bacteroides and clostridia compared to galacto-oligosaccharides with fructan. The weight ratio transgalacto-oligosaccharide : fructo-oligosaccharide : pectin degradation product is preferably (20 to 2) : 1 : (1 to 20), more preferably (12 to 7) : 1 : (1 to 3).

Preferably, the composition comprises of 80 mg to 2 g non-digestible oligosaccharides per 100 ml, more preferably 150 mg to 1.50 g, even more preferably 300 mg to 1 g. Based on dry weight, the composition preferably comprises 0.25 wt.% to 5.5 wt.%, more preferably 0.5 wt.% to 4 wt.%, even more preferably 1.5 wt.% to 3 wt.%. A lower amount of non-digestible oligosaccharides will be less effective in stimulating the beneficial bacteria in the microbiota, whereas a too high amount will result in side-effects of bloating and abdominal discomfort.

### Sphingophospholipids and its degradation products

The present composition comprises sphingophospholipids and/or one of its degradation products. The term sphingophospholipid as in the present invention relates to a type of sphingolipid consisting of a sphingoid base bonded to one fatty acid via an amide bond and, via an oxygen, to one polar headgroup comprising a phosphate (phosphate, phosphocholine, phosphoserine, phosphoethanolamine, phosphoglycerol, or phosphoinositol). In another embodiment of the present invention a degradation product derived from sphingophospholipid, particularly ceramides, lysosphingophospholipid, sphingoid phosphate and/or the free sphingoid base, is present in the composition. Sphingoid bases in the present invention are preferably 2-amino-1,3-dihydroxy-alkanes or alkenes with *(2S,3R)-erythro* stereochemistry, with a chain length of 14 atoms or higher. Sphingoid bases may comprise three instead of two hydroxyl groups. The aliphatic chains may be mono- or poly-unsaturated (in the cis- or trans-configuration) or saturated. The aliphatic chain may be branched. Sphingoid bases include sphingosine, sphinganine, phytosphingosine and dihydrophytosphingosine. Preferably the composition comprises sphingomyelin. Sphingomyelin is ceramide phosphorylcholine. Ceramide consists of a sphingoid base linked to a fatty acid via an amide bond.

Upon oral consumption, sphingophospholipids and/or degradation products thereof, in particular lysosphingolipid, ceramide, sphingoid phosphate and/or sphingoid base, has a reducing effect on unfavourable bacteria in the intestinal microbiota, such as firmicutes, especially clostridia. Having both a stimulator of beneficial bacteria and an inhibitor of unfavourable bacteria present will improve the composition of the microbiotia and/or keep the microbiota well balanced in a much more effective way.

Preferably, the composition comprises 10 to 200 mg sphingomyelin per 100 ml, more preferably 20 mg to 150 mg, even more preferably 60 mg to 100 mg. Based on fat, the composition preferably comprises 0.25 wt.% to 5.5 wt.%, more preferably 0.5 wt.% to 4 wt.%, even more preferably 1.5 wt.% to 3 wt.%. Based on dry weight, the composition preferably comprises 0.07 wt.% to 1.5 wt.%, more preferably 0.15 wt.% to 1.1 wt.%, even more preferably 0.5 wt.% to 0.8 wt.%. A lower amount will be less effective, whereas a higher amount will leave less room for the advantageous essential and longchain fatty acids. A suitable source of sphingophospholipid is milk fat, more suitable butter milk fat or butter serum fat, egg yolk and/or soy lecithin.

Preferably the weight ratio between non-digestible oligosaccharides and sphingomyelin is between 0.4 and 200, more preferably between 1 and 75, even more preferably between 3 and 15. Having this ratio present in the composition ensures a balance between the stimulating effect on beneficial bacteria in the microbiota and a reducing effect on unfavourable bacteria in the microbiota.

### Other lipids

Herein LA refers to linoleic acid (18:2 n6); ALA refers to alpha-linolenic acid (18:3 n3); LC-PUFA refers to long chain polyunsaturated fatty acids and/or acyl chains comprising at least 20 carbon atoms in the fatty acyl chain and with 2 or more unsaturated bonds; DHA refers to docosahexaenoic acid (22:6, n3); EPA refers to eicosapentaenoic acid (20:5 n3); ARA refers to arachidonic acid (20:4 n6); DPA refers to docosapentaenoic acid (22:5 n3).

LA preferably is present in a sufficient amount in order to promote a healthy growth and development, yet in an amount as low as possible to prevent occurrence of obesity later in life. The composition therefore preferably comprises less than 15 wt.% LA based on total fatty acids, preferably between 5 and 14.5 wt.%, more preferably between 6 and 12 wt.%. Preferably ALA is present in a sufficient amount to promote a healthy growth and development. The present composition therefore preferably comprises at least 1.0 wt.% based on total fatty acids. Preferably the composition comprises at least 1.6 wt.% ALA based on total fatty acids, more preferably at least 2.0 wt.%. Preferably the composition comprises less than 10 wt.% ALA, more preferably less than 5.0 wt.% based on total fatty acids. The weight ratio LA/ALA should be well balanced in order to prevent obesity, especially central obesity, while at the same time ensuring a normal growth and development. Therefore, the present composition preferably comprises a weight ratio of LA/ALA between 2 and 15, more preferably between 2 and 7, more preferably between 3 and 6, even more preferably between 4 and 5.5, even more preferably between 4 and 5.

Preferably the present composition comprises LC-PUFA, since LC-PUFA reduce obesity later in life, more preferably central obesity. More preferably, the present composition comprises n-3 LC-PUFA, even more preferably EPA, DPA and/or DHA, even more preferably DHA. It was found that these n-3 LC-PUFA decrease obesity. Since a low concentration of DHA, DPA and/or EPA is already effective and normal growth and development are important, the content of n-3 LC-PUFA in the present composition, preferably does not exceed 15 wt.% of the total fatty acid content, preferably does not exceed 10 wt.%, even more preferably does not exceed 5 wt.%. Preferably the present composition comprises at least 0.2 wt.%, preferably at least 0.5 wt.%, more preferably at least 0.75 wt.%, n-3 LC-PUFA of the total fatty acid content. The weight ratio n-6 LC-PUFA / n-3 LC-PUFA in the present composition is preferably low in order to prevent obesity later in life. Preferably the composition comprises a weight ratio of n-6 LC-PUFA / n-3 LC-PUFA below 1.5, more preferably below 1.0, even more preferably below 0.6. LC-PUFA have the further advantage that they facilitate the adhesion of beneficial bacteria, thereby further improving the microbiota and/or maintaining a well balanced microbiota.

Preferably the present composition comprises cholesterol and/or cholesterylester. The simultaneous presence of sphingophospholipid and cholesterol and/or cholesterylester in the composition delays the digestion of the sphingophospholipid in the small intestine, thereby increasing the amounts of sphingophospholipid which reaches the large intestine and hence the activity of sphingophospholipid and/or degradation products thereof against unfavourable bacteria in the intestinal microbiota. The present composition preferably comprises at least 0.005 wt.% cholesterol and/or cholesterylester based on total fat, more preferably at least 0.01 wt.%, more preferably at least 0.05 wt.%, even more preferably at least 0.1 wt.%. Preferably the amount of cholesterol and/or cholesteryester does not exceed 10 wt.% based on total lipid, more preferably does not exceed 5 wt.%, even more preferably does not exceed 1 wt.% of total lipid. Most preferably the amount of cholesterol is 0.5 to 0.7 wt.% based on total lipid.

### Nutritional compositions

The present composition is preferably orally administered. Degradation of the sphingophospholipid by the digestive enzymes is necessary to improve its action against unfavourable bacteria in the intestinal microbiota. The present composition is preferably a nutritional formula, preferably an infant formula. The present composition comprising non-digestible carbohydrates and sphingophospholipids and/or degradation products thereof is not human milk.

The present composition can advantageously be applied as a complete nutrition for infants. The present composition preferably comprises lipid, protein and digestible carbohydrate and is preferably administered in liquid form. The present invention includes dry food (e.g. powders) which is accompanied with instructions as to mix said dry food mixture with a suitable liquid (e.g. water).

The present invention advantageously provides a composition wherein the lipid provides 5 % to 50 % of the total calories. The present invention advantageously provides a composition wherein the protein provides 5 % to 50 % of the total calories. The present invention advantageously provides a composition wherein the digestible carbohydrate provides 15 % to 90% of the total calories. The present invention advantageously provides a composition wherein the lipid provides 5 % to 50 % of the total calories, the protein provides 5 % to 50 % of the total calories, and the digestible carbohydrate provides 15 % to 90% of the total calories. Preferably, in the present composition the lipid provides 35 % to 50% of the total calories, the protein provides 7.5 % to 12.5% of the total calories, and the digestible carbohydrate provides 40 % to 55% of the total calories. For calculation of the percentage of total calories for the protein, the total of energy provided by the proteins, peptides and amino acids needs to be taken into account. Total calories relates to the sum of the calories provided by the lipid, the digestible carbohydrate and the protein.

The protein used in the nutritional preparation is preferably selected from the group consisting of non-human animal proteins (preferably milk proteins), vegetable proteins (preferably soy protein and/or rice protein), free amino acids and mixtures thereof. The present composition preferably contains casein, whey, hydrolysed casein and/or hydrolysed whey protein. Preferably the protein comprises intact proteins, more preferably intact bovine whey proteins and/or intact bovine casein proteins. As the present composition is suitably used to reduce the allergic reaction in an infant, the protein of is preferably selected from the group consisting of hydrolysed milk protein.

The present composition preferably contains digestible carbohydrate selected from the group consisting of sucrose, lactose, glucose, fructose, corn syrup solids, starch and maltodextrins, more preferably lactose.

Preferably the present composition comprises nucleotides and/or nucleosides, more preferably nucleotides. Preferably, the composition comprises cytidine 5'-monophospate, uridine 5'-monophospate, adenosine 5'-monophospate, guanosine 5'-monophospate, and/or inosine 5'-monophospate, more preferably cytidine 5'-monophospate, uridine 5'-monophospate, adenosine 5'-monophospate, guanosine 5'-monophospate, and inosine 5'-monophospate. Preferably the composition comprises 5 to 100 mg, more preferably 5 to 50 mg, most preferably 10 to 50 mg nucleotides and/or nucleosides per 100 g dry weight of the composition. The presence of nucleotides and/or nucleotides advantageously lowers the ratio of the bacteroides, porphyromonas and/or prevotella group to bifidobacteria.

The nucleotides and/or nucleosides are deemed to act synergistically with the other ingredients of the present composition.

Stool irregularities, e.g. hard stools, insufficient stool volume, diarrhoea, is an important problem in babies. It was found that stool problems may be reduced by administering the present composition in a liquid form with an osmolality between 50 and 500 mOsm/kg, more preferably between 100 and 400 mOsm/kg. The reduced stool irregularities enhance the colonization and development of a healthy intestinal microbiota.

In view of the above, it is also important that the liquid food does not have an excessive caloric density, however still provides sufficient calories to feed the subject. Hence, the liquid food preferably has a caloric density between 0.1 and 2.5 kcal/ml, even more preferably a caloric density of between 0.5 and 1.5 kcal/ml, most preferably between 0.6 and 0.8 kcal/ml.

### Applications

The present composition comprising sphingophospholipids and/or degradation products thereof, preferably sphingomyelin, and a non-digestible carbohydrate is particularly suitable for use in a human subject having an age below 36 months for use in normalising the composition of the intestinal microbiota. The present composition is particularly suitable for use in a human subject having an age below 36 month for use in normalising the composition of the intestinal microbiota when said human subject has reached an age above 36 months.

Normalising the intestinal microbiota in the present human subjects below 36 months of age relates to normalising of the composition of the intestinal microbiota towards the composition found in the intestinal microbiota of healthy human milk fed human subjects with an age below 36 months. The use in a human subject having an age below 36 months for use in normalising the composition of the intestinal microbiota when said human subject has reached an age above 36 months, relates to normalising of the composition of the intestinal microbiota towards the composition found in the intestinal microbiota of healthy subjects of the same age which have been human milk fed in the past when they were of an age below 36 months.

The present composition comprising sphingophospholipid and/or degradation products thereof, preferably sphingomyelin, and a non-digestible carbohydrate is particularly suitable for use in a human subject having an age above 36 months for use in normalising the composition of the intestinal microbiota. Normalising the intestinal microbiota in human subjects above 36 months of age relates to normalising of the composition of the intestinal microbiota towards the composition found in the intestinal microbiota of healthy human subjects of normal weight. The present composition comprising sphingophospholipid and/or degradation products thereof, preferably sphingomyelin, and a non-digestible carbohydrate is particularly suitable for use in an obese human subject for use in normalising the composition of the intestinal microbiota. Normalising the intestinal microbiota in obese human subjects relates to normalising of the composition of the intestinal microbiota towards the composition found in the intestinal microbiota of healthy human subjects with normal weight.

The present composition comprising sphingophospholipid and/or degradation products thereof, preferably sphingomyelin, and a non-digestible carbohydrate is particularly suitable for use in a human subject using or having recently used antibiotics for use in normalising the composition of the intestinal microbiota. The term 'recently' in the context of the present invention refers to ending the use of antibiotics less than 2 months ago, for example less than 1 month ago or less than 1 week ago. Normalising the intestinal microbiota in human subjects using or having recently used antibiotics relates to normalising of the composition of the intestinal microbiota towards the composition found in the intestinal microbiota of said human subjects compared to the period before antibiotic use or compared to the composition found in the intestinal microbiota.

Normalising the intestinal microbiota in the present invention in particular relates to an increased prebiotic index, a decreased ratio of *Clostridia*/*total* bacteria, a decreased ratio *Clostridium difficile*/total bacteria, a decreased ratio *Clostridum difficile*/*total* clostridia, an increased ratio lactobacilli/total bacteria, an increased ratio *Bifidobacteria*/total bacteria, a decreased ratio Firmicutes/Bacteroidetes, a decreased ratio *Clostridici*/*Bacteroides,* a decreased ratio *Enterobacteriae*/total bacteria. Increase or decrease is with respect to the ratio present in the human subject prior to ingestion of the present composition, or to a control group not taking this composition. The prebiotic index (PI) relates to the sum of: (*Bifidobacteria*/total bacteria) + (*Lactobacilli*/total bacteria)- (*Bacteroides*/total bacteria) -(*Clostridia*/total bacteria), see Palframan et al, 2003, Lett Appl Microbiol 37:281-284. Preferably, also the ratio of *Eubacterium rectale*/total bacteria may be added to this, more particularly for adults. *Eubacterium rectale* produces butyrate which is advantageous for the gut barrier in adults.

The intestinal microbiota relates in particular to the microbiota composition of the colon. The composition of the intestinal microbiota can be determined on the level phylum, class, family, genus and/or species by methods known in the art, including FISH, real time PCR and micro-arrays, using specific probes and/or primers known in the art.

The present composition is especially advantageous for infants born in the hospital, since they are at risk of being colonised by unfavourable bacteria deriving from the hospital environment. The present composition is even more advantageous for infants delivered via caesarean section, since these infants lack a proper inoculation with beneficial bacteria deriving from the mother, while at the same time they are at risk of being colonised by unfavourable bacteria deriving from the hospital environment.

Since administration of the present composition to human subjects below 36 months of age has the advantageous effects on microbiota as described above, the present composition is preferably subsequently used for the prevention and/or treatment of obesity and/or adiposity, more preferably visceral obesity, more preferably when said human subject has reached an age above 36 months. Subsequently the present composition preferably is used to prevent secondary disorders resulting from visceral obesity, such as type 2 diabetes, fasting hyperglycaemia, insulin resistance, visceral adiposity, hyperinsulinemia, hypertension, cardiovascular disease, cerebrovascular disease, artherosclerose, dyslipidaemia, hyperuricaemia, fatty liver, osteoarthritis and sleep apnoea when said human subject has reached an age above 36 months. In one embodiment obesity, adiposity and visceral obesity, refer to said conditions as the consequence of unbalanced intestinal microbiota. Preferably unbalanced microbiota refers to an increased ratio of Firmicutes/Bacteroidetes, an increased ratio *Clostridium difficile*/total bacteria, and/or a decreased prebiotic index compared to healthy lean subjects and/or to breast-fed infants.

Since administration of the present composition to human subjects below 36 months of age has the advantageous effects on microbiota as described above, the present composition is preferably subsequently used for the treatment and/or prevention of gastrointestinal infection, diarrhoea and/or gastrointestinal inflammation, more preferably when said human subject has reached an age above 36 months.

Since administration of the present composition to human subjects below 36 months of age has the advantageous effects on microbiota as described above, the present composition is preferably subsequently used for improving the immune system and/or the gastro-intestinal barrier function, more preferably when said human subject has reached an age above 36 months.

Since administration of the present composition to human subjects below 36 months of age has the advantageous effects on microbiota as described above, the present composition is preferably subsequently used for treatment and/or prevention of at least one disorder selected form the group consisting of allergy, asthma, atopic dermatitis, eczema, systemic infections, urinary tract infections, otitis and respiratory infections, more preferably when said human subject has reached an age above 36 months.

The present invention aims to normalize intestinal microbiota in human subjects preferably with an age above 36 months, preferably an age above 8 years, more preferably an age above 15 years, most preferably above 18 years.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### EXAMPLES

### Example 1

Pooled fresh faeces obtained from 4-5 healthy adults was used to perform fermentation studies as described in Appendix I of WO 2004/000340. The experiments were performed in duplo. The faeces, diluted 1:5 into McBain and Farlane medium, was put into dialysis tubes and suspended in bottles with buffer under anaerobic conditions and was first incubated at 37 °C for 16 h for reasons of adaptation before inoculating them with the substrates according to the table below at t=0.

The test substrates were added once a day (at t=0, 24, and 48 hours). Buffer was refreshed once a day. A sample was taken at t=0 and t=72. This relatively long period was used since shifts in bacterial populations are relatively slow compared to the shifts in short chain acids formed.

**Table 1: Additions to the dialysis tube comprising diluted faeces**

| Number | Sphingosine | GOS | Inulin |
|---|---|---|---|
| 1 | 300 µg | 0 | 0 |
| 2 | 0 | 103.7 mg | 11.5 mg |
| 3 | 300 µg | 103.7 mg | 11.5 mg |

As a source of GOS Vivinal GOS (Borculo Domo, The Netherlands was used). As a source of inulin RaftilineHP (Orafti, Belgium) was used.

D-sphingosine was added to the dialysis tubes instead of sphingophospholipid such as sphingomyelin, since degradation of sphingophospholipids in the small intestine gives D-sphingosine. As a source of D-sphingosine D sphingosine from bovine brain (Fluka Sigma Aldrich Chemie) was used.

The sphingosine was dissolved in ethanol (stock 10 mg/ml). 30 µl of a stock of 10 mg/ml was added to the dialysis tube.

The samples were analysed by Fluorescence *In Situ* Hybridisation (FISH) as described in Franks, A.H., et al., Appl. Environ. Microbiol., 1998. 64(9): p. 3336-3345. For determination of the Bacteroides-Prevotella group the Bac303 probe was used (Manz, W., et al., Microbiology, 1996. 142 (Pt 5): p. 1097-106). For determination of Clostridia the Chis150 and Clit135 probe were used (Franks, A.H., et al., Appl. Environ. Microbiol., 1998. 64(9): p. 3336-3345). For determination of the *Eubacterium rectale* and *Clostridium coccoides* group the Erec482 probe was used (Franks, A.H., et al., Appl. Environ. Microbiol., 1998. 64(9): p. 3336-3345). For Bifidobacteria determination the Bif164 probe was used (Langendijk, P., et al., Appl. Environ. Microbiol., 1995. 61(8): p. 3069-3075). For determination of Lactobacilli and Enterococci the Lab158 probe was used (Harmsen, H.J.M., et al., Microbial Ecology in Health and Disease, 1999. Volume 11: p. 3-12). For determination of the enterobacteria the Ec1531 probe was used (Poulsen, L.K., et al., Infect Immun, 1994. 62(11): p. 5191-4). Total number of bacteria was determined by DAPI staining. The ratio Firmicutes/Bacteroidetes was set at the percentage of bacteria detected by the Chis150, Clit135, Erec482 and Lab158 probe, divided by the percentage of bacteria divided by the Bac303 probe. The bacteria detected by the Erec482 group are usually the largest group of Firmicutes in the human intestine.

### Results:

The Bac303, Chis150, Clit135, Ec1351 and Lab158 probe all showed signals below the detection limit of FISH, being <10⁶ cells per gram of sample or a relative abundance lower than 1 in 1000 cells (0.1%). In case of the Bac303 the samples were stored under poor conditions, but a faint signal was observed in the samples to which GOS and inulin was added. The FISH results of the Bif164 and Erec482 probes are shown in Table 2.

**Table 2: FISH results for Bif164 and Erec482 probes.**

| Number | Time (h) | Bif164 % total bacteria (SD) | Erec482 % total bacteria (SD) |
|---|---|---|---|
| | t=0 | 3.15 | 33.99 |
| 1 | t=72 | 5.93 | 17.45 |
| 2 | t=72 | 7.05 | 21.84 |
| 3 | t=72 | 10.13 | 12.83 |

These results are indicative of an increase in bifidobacteria when non-digestible carbohydrates more particular GOS and/or inulin, are fermented or when sphingosine is present (being the degradation product of sphingophospholipid after digestion in the small intestine) and an even synergistically further increase when both components are present.

These results are indicative that the Clostridia and Eubacteria, being the largest group of intestinal Firmicutes, decrease when non-digestible carbohydrates more particular GOS and/or inulin are fermented or when sphingosine is present. When both components are present a further improved effect is observed.

These results are indicative for improvement of increased prebiotic index, a decreased ratio of clostridia/total bacteria, an increased ratio *Bifidobacteria*/total bacteria, a decreased ratio Firmicutes/Bacteroidetes, and a decreased ratio *Clostridia*/*Bacteroides* in the large intestinal microbiota upon consumption of non-digestible carbohydrates, in particular GOS and/or inulin, and sphingophospholipids.

### Example 2: Infant nutrition

Infant nutrition comprising lipid providing 48% of the total calories, protein providing 8% of the total calories and a digestible carbohydrate providing 44% of the total calories;
(i) the lipid comprising about 1.4 wt.% sphingomyelin and about 4 wt.% cholesterol based on total lipid;
(ii) the digestible carbohydrate comprising 50.9 gram lactose/100 gram powder, and further comprising as non-digestible carbohydrates 5.22 g beta-galacto-oligosaccharides with DP 2-6 and 0.58 g fructo-oligosaccharides with DP 7-60 per 100 g powder;
(iii) the protein comprising cow milk protein, including casein.

The composition comprises vitamins, trace elements and minerals according to EU directives. The label of the package of this infant nutrition indicates that the nutrition aims to improve the intestinal flora, increase the number of intestinal bifidobacteria, decrease the number of intestinal pathogens and/or to keep a well balanced intestinal flora.

## Claims

1. Use of a nutritional composition comprising
A sphingophospholipid, preferably sphingomyelin, and/or at least one sphingophospholipid degradation product selected from the group consisting of ceramide, lysosphingophospholipid, sphingoid phosphate, and free sphingoid base, and
B at least one non-digestible carbohydrate selected from the group consisting of fructo-oligosaccharides, galacto-oligosaccharides, gluco-oligosaccharides, arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides
for the manufacture of a composition for normalising the intestinal microbiota in a human subject towards the microbiota found in healthy lean subjects and/or to breast-fed infants.

2. Use according to claim 1 wherein said human subject has an age below 36 months.

3. Use according to claim 1 or 2 wherein the microbiota remains normalised when said human subject has reached an age above 36 months.

4. Use according to any of the preceding claims wherein the composition of the intestinal microbiota is normalised with respect to at least one selected from the group consisting of an increased prebiotic index, a decreased ratio of clostridia/total bacteria, a decreased ratio *Clostridium difficile*/total bacteria, a decreased ratio *Clostridium difficile*/*Clostridia,* an increased ratio *Lactobacilli*/total bacteria, an increased ratio *Bifidobacteria*/total bacteria, a decreased ratio Firmicutes/Bacteroidetes, a decreased ratio *Clostridici*/*Bacteroides* and a decreased ratio *Enterobacteria*/total bacteria

5. Use according any of the preceding claims for the prevention of obesity and/or adiposity, more preferably for use in a human subject below 36 months of age and for the prevention of obesity and/or adiposity when said human subject has reached an age above 36 months.

6. Use according to claim 5 for the prevention of a disorder selected from the group consisting of type 2 diabetes, fasting hyperglycaemia, insulin resistance, visceral adiposity, hyperinsulinemia, hypertension, cardiovascular disease, cerebrovascular disease, artherosclerose, dyslipidaemia, hyperuricaemia, fatty liver, osteoarthritis and sleep apnoea.

7. Use according to any of claims 1-4 for the treatment and/or prevention of gastrointestinal infection, diarrhoea and/or gastrointestinal inflammation, allergy, asthma, atopic dermatitis, eczema, systemic infections and respiratory infections.

8. Use according to any of the preceding claims wherein the composition comprises at least 0.25 wt.% sphingophospholipid, preferably sphingomyelin, and/or at least one sphingophospholipid degradation product selected from the group consisting of ceramide, lysosphingophospholipid, sphingoid phosphate, and free sphingoid base based on lipid.

9. Use according to any of the preceding claims wherein at least two non-digestible carbohydrates are selected.

10. Use according to any of the preceding claims wherein the composition comprises at least 0.25 wt.% g non-digestible carbohydrate based on dry weight of the composition.

11. Use according to any of the preceding claims wherein the composition comprises lipid providing 35 to 50 % calories based on total calories, digestible carbohydrate providing 40 to 55 % calories based on total calories and protein providing 7.5 to 12.5 % calories based on total calories of the composition.

12. Use according to any of the preceding claims, wherein the composition comprises a weight ratio of sphingophospholipid and/or degradation products thereof to non-digestible carbohydrate between 0.4 and 200.

## Patentansprüche

1. Verwendung einer Ernährungszusammensetzung, umfassend
A Sphingophospholipid, bevorzugt Sphingomyelin, und/oder mindestens ein Sphingophospholipid-Abbauprodukt ausgewählt aus der Gruppe bestehend aus Ceramid, Lysosphingophospholipid, Sphingoidphosphat und freier Sphingoid-Base, und
B mindestens ein unverdauliches Kohlenhydrat ausgewählt aus der Gruppe bestehend aus Fructooligosacchariden, Galactooligosacchariden, Glucooligosacchariden, Arabinooligosacchariden, Mannanoligosacchariden, Xylooligosacchariden, Fucooligosacchariden, Arabinogalactooligosacchariden, Glucomannooligosacchariden, Galactomannooligosacchariden, Sialinsäure umfassenden Oligosacchariden und Uronsäureoligosacchariden
zur Herstellung einer Zusammensetzung zum Normalisieren der Darmmikrobiota in einer menschlichen Person in Richtung der Mikrobiota, die in gesunden schlanken Personen gefunden werden und/oder in Richtung von gestillten Babies.

2. Verwendung nach Anspruch 1, wobei die menschliche Person ein Alter von unter 36 Monaten aufweist.

3. Verwendung nach Anspruch 1 oder 2, wobei die Mikrobiota normalisiert bleiben, wenn die menschliche Person ein Alter von über 36 Monaten erreicht hat.

4. Verwendung nach einem beliebigen der voranstehenden Ansprüche, wobei die Zusammensetzung der Darmmikrobiota normalisiert ist bezüglich mindestens einem ausgewählt aus der Gruppe bestehend aus einem erhöhten präbiotischen Index, einem verringerten Verhältnis von Clostridia/Gesamtbakterien, einem verringerten Verhältnis von *Clostridium difficile*/Gesamtbakterien, einem verringerten Verhältnis von *Clostridium difficile*/*Clostridia,* einem erhöhten Verhältnis von *Lactobacilli*/Gesamtbakterien, einem erhöhten Verhältnis von *Bifidobacteria*/Gesamtbakterien, einem verringerten Verhältnis von Firmicutes/Bacteroidetes, einem verringerten Verhältnis von *Clostridia*/*Bacteroides,* und einem verringerten Verhältnis von *Enterobacteria*/Gesamtbakterien.

5. Verwendung nach einem beliebigen der voranstehenden Ansprüche zur Vorbeugung von Obesitas und/oder Adipositas, stärker bevorzugt zur Verwendung in einer menschlichen Person unter 36 Monaten alt und zur Vorbeugung von Obesitas und/oder Adipositas, wenn die menschliche Person ein Alter von über 36 Monaten erreicht hat.

6. Verwendung nach Anspruch 5 zur Vorbeugung einer Störung ausgewählt aus der Gruppe bestehend aus Diabetes Typ 2, Nüchtern-Hyperglykämie, Insulinresistenz, viszerale Adipositas, Hyperinsulinämie, Hypertonie, Herz-Kreislauf-Erkrankung, zerebrovaskuläre Krankheit, Arteriosklerose, Dyslipidämie, Hyperurikämie, Fettleber, Arthrose und Schlafapnoe.

7. Verwendung nach einem beliebigen der Ansprüche 1-4 zur Behandlung und/oder Vorbeugung von gastrointestinalem Infekt, Diarrhö und/oder Entzündung des Magen-Darm-Trakts, Allergie, Asthma, atopischer Dermatitis, Ekzem, systemischen Infektionen und Atemwegsinfektionen.

8. Verwendung nach einem beliebigen der voranstehenden Ansprüche, wobei die Zusammensetzung mindestens 0,25 Gew.-% Sphingophospholipid, bevorzugt Sphingomyelin, und/oder mindestens ein Sphingophospholipid-Abbauprodukt ausgewählt aus der Gruppe bestehend aus Ceramid, Lysosphingophospholipid, Sphingoidphosphat, und freier Sphingoid-Base basierend auf Lipid umfasst.

9. Verwendung nach einem beliebigen der voranstehenden Ansprüche, wobei mindestens zwei unverdauliche Kohlenhydrate ausgewählt sind.

10. Verwendung nach einem beliebigen der voranstehenden Ansprüche, wobei die Zusammensetzung mindestens 0,25 Gew.-% g unverdauliches Kohlenhydrat basierend auf dem Trockengewicht der Zusammensetzung umfasst.

11. Verwendung nach einem beliebigen der voranstehenden Ansprüche, wobei die Zusammensetzung 35 bis 50 % der Gesamtkalorien an Lipid, 40 bis 55 % der Gesamtkalorien an verdauliche Kohlenhydrat und 7.5 bis 12.5 % der Gesamtkalorien an Protein umfasst.

12. Verwendung nach einem beliebigen der voranstehenden Ansprüche, wobei die Zusammensetzung ein Gewichtsverhältnis von Sphingophospholipid und/oder Abbauprodukt davon an unverdaulichenKohlenhydrat zwischen 0,4 und 200 aufweist.

## Revendications

1. Utilisation d'une composition nutritionnelle comprenant
A. un sphingophospholipide, de préférence la sphingomyéline, et/ou au moins un produit de dégradation de sphingophospholipide choisi dans le groupe consistant en céramide, lysosphingophospholipide, sphingoïde phosphate et base sphingoïde libre, et
B au moins un glucide non-digestible choisi dans le groupe consistant en fructo-oligosaccharides, galacto-oligosaccharides, gluco-oligosaccharides, arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, acide sialique comprenant des oligosaccharides et oligosaccharides de l'acide uronique
pour la fabrication d'une composition pour normaliser le microbiote intestinal chez un sujet humain vers le microbiote trouvé chez des sujets minces sains et/ou des enfants nourris au sein.

2. Utilisation selon la revendication 1, dans laquelle ledit sujet humain est âgé de moins de 36 mois.

3. Utilisation selon la revendication 1 ou 2, dans laquelle le microbiote reste normalisé lorsque ledit sujet humain a atteint un âge de plus de 36 mois.

4. Utilisation selon l'une quelconque des revendications précédentes, dans laquelle la composition du microbiote intestinal est normalisée concernant au moins l'un choisi dans le groupe consistant en un indice prébiotique accru, un rapport clostridia/bactéries totales réduit, un rapport *Clostridium difficile*/*bactéries* totales réduit, un rapport *Clostridium difficile*/*Clostridia* réduit, un rapport *Lactobacilli*/bactéries totales accru, un rapport *Bifidobacteria*/bactéries totales accru, un rapport Firmicutes/Bacteroidetes réduit, un rapport *Clostridia*/*Basteroides* réduit et un rapport *Enterobacterie*/bactéries totales réduit.

5. Utilisation selon l'une quelconque des revendications précédentes pour la prévention de l'obésité et/ou de l'adiposité, plus préférentiellement pour l'utilisation chez un sujet humain âgé de moins de 36 mois et pour la prévention de l'obésité et/ou de l'adiposité lorsque ledit sujet humain a atteint un âge de plus de 36 mois.

6. Utilisation selon la revendication 5 pour la prévention d'un trouble choisi dans le groupe consistant en le diabète de type 2, l'hyperglycémie à jeun, la résistance à l'insuline, l'adiposité viscérale, l'hyperinsulinémie, l'hypertension, les maladies cardiovasculaires, les maladies cérébrovasculaires, l'athérosclérose, la dyslipidémie, l'hyperuricémie, la stéatose du foie, l'arthrose et l'apnée du sommeil.

7. Utilisation selon l'une quelconque des revendications 1 à 4 pour le traitement et/ou la prévention d'une infection gastro-intestinale, de la diarrhée et/ou d'une inflammation gastro-intestinale, d'une allergie, de l'asthme, de la dermatite atopique, de l'eczéma, des infections systémiques et des infections respiratoires.

8. Utilisation selon l'une quelconque des revendications précédentes, dans laquelle la composition comprend au moins 0,25 % en poids de sphingophospholipide, de préférence, de sphingomyéline, et/ou d'au moins un produit de dégradation de sphingophospholipide choisi dans le groupe consistant en céramide, lysosphingophospholipide, sphingoïde phosphate et base sphingoïde libre sur la base des lipides.

9. Utilisation selon l'une quelconque des revendications précédentes, dans laquelle au moins deux glucides non-digestibles sont choisis.

10. Utilisation selon l'une quelconque des revendications précédentes, dans laquelle la composition comprend au moins 0,25 % en poids de glucides non-digestibles sur la base du poids sec de la composition.

11. Utilisation selon l'une quelconque des revendications précédentes, dans laquelle la composition comprend des lipides fournissant 35 à 50 % de calories sur la base des calories totales, des glucides digestibles fournissant 40 à 55 % de calories sur la base des calories totales et des protéines fournissant 7,5 à 12,5 % de calories sur la base des calories totales de la composition.

12. Utilisation selon l'une quelconque des revendications précédentes, dans laquelle la composition comprend un rapport en poids de sphingophospholipide et/ou de produits de dégradation de celui-ci aux glucides non-digestibles compris entre 0,4 et 200.
